# EUROPÄISCHE PATENTSCHRIFT

(11) **EP 0 808 158 B1**
(45) Veröffentlichungstag und Bekanntmachung des Hinweises auf die Patenterteilung: **08.03.2000**
(21) Anmeldenummer: 96900974.5
(22) Anmeldetag: 25.01.1996
(51) Int. Cl.: A61K 9/70, A61L 15/38, A61L 15/44

(54) **ARZNEIFORM ZUR ABGABE VON WIRKSTOFFEN AN WUNDEN**
MEDICAMENT FORM FOR THE DELIVERY OF ACTIVE SUBSTANCES TO WOUNDS
FORME MEDICAMENTEUSE POUR APPORT DE PRINCIPES ACTIFS A DES BLESSURES

(30) Priorität: 02.02.1995 DE 19503336
(43) Veröffentlichungstag der Anmeldung: 26.11.1997
(73) Patentinhaber: LTS Lohmann Therapie-Systeme AG, 56626 Andernach (DE)
(72) Erfinder: ROREGER, Michael, D-56567 Neuwied (DE)
(74) Vertreter: Flaccus, Rolf-Dieter, Dr.
(86) Internationale Anmeldenummer: EP9600295
(87) Internationale Veröffentlichungsnummer: WO9623488

(56) Entgegenhaltungen:
- EP-A- 0 460 588
- DE-A- 3 139 089
- DE-A- 3 606 265

## Beschreibung

In der Wundbehandlung müssen Wirkstoffe, die mit der Wundoberfläche bzw. dem Wundgrund in direkten Kontakt kommen sollen oder müssen, mittels Arzneiformen verabreicht werden, die aufgrund ihrer Konsistenz auch auf sehr unebene Oberflächen lückenlos aufgebracht werden können. Dies geschieht üblicherweise mit Hilfe von Lösungen, Pulvern, Pudern, Sprays, halbfesten Zubereitungen wie Salben, Cremes und Gelen. Die Nachteile dieser Arzneiformen werden insbesondere dann deutlich, wenn hochwirksame Substanzen in geringer Menge exakt und reproduzierbar dosiert werden müssen, oder wenn für die Therapie eine kontrollierte Freisetzung aus der Arzneiform zur Aufrechterhaltung einer gleichmäßigen Wirkstoffkonzentration in der Wunde über einen bestimmten Zeitraum hinweg wünschenswert ist.

Wundauflagen, die aufgrund ihres Aufbaus und ihrer Struktur mit Wirkstoffen beladen werden und diese verzögert oder kontrolliert freisetzen, sind beispielsweise in der US 5,098,417, in der EP 49 177 oder in der DE-AS 11 90 608 beschrieben. Diese Wundauflagen weisen den Nachteil auf, daß sie insbesondere bei tiefen Wunden nicht mit dem Wundgrund in Kontakt kommen. Die Funktionsfähigkeit solcher Wundauflagen ist zudem in starkem Maße von der Interaktion mit Wundflüssigkeit abhängig, da die Freisetzung nur durch Diffusion des Wirkstoffs an der Grenzfläche zwischen Wundauflage und Wundflüssigkeit oder durch Erosion des Wirkstoffs aus der Wundauflage nach Flüssigkeitsaufnahme und Quellung des Trägermaterials erfolgen kann. Da die Flüssigkeitsverhältnisse in Wunden individuell sowie in Abhängigkeit von Wundtyp und Phase der Wundheilung stark schwanken, können vergleichbare und reproduzierbare Freisetzungskinetiken mit solchen Wundauflagen in vivo nicht erreicht werden.

Der vorliegenden Erfindung liegt die Aufgabe zugrunde, eine Arzneiform anzugeben, die einerseits eine genaue und reproduzierbare Wirkstoffdosierung sowie eine verläßliche Steuerung der Wirkstofffreisetzung ermöglicht, und die andererseits entsprechend den eingangs genannten traditionellen Arzneiformen auch bei tiefen Wunden an unebene Wundflächen anpaßbar und in Kontakt zu diesen bringbar ist. Überraschenderweise wurde die Lösung in einer Einmaldosen-Arzneiform gefunden, welche dadurch gekennzeichnet ist, daß diese als auf einem filmartigen flexiblen Substrat angeordnete vorportionierte plastische kohärente Masse mit definiertem Wirkstoffgehalt in homogener Verteilung ausgebildet ist und auf der dem Substrat abgewandten Seite eine an die Wundoberfläche andrückbare Kontaktfläche für eine lückenlose Wirkstoffabgabe an die kontaktierte Wundoberfläche aufweist.
Weitere Ausgestaltugen der Arzneiform sind entsprechend den Merkmalen der Unteransprüche vorgesehen.

Herkömmliche verformbare, wirkstoffabgebende Arzneiformen, die nach Applikation in die Wunde flächenförmige Strukturen ausbilden, wie beispielsweise Gele, Salben, Cremes oder auch flüssige Mehrkomponentensysteme, die nach dem Zusammengeben in der Wunde unter Verfestigung miteinander reagieren, gehören nach der Fachterminologie zu den sogenannten Mehrfachdosen-Arzneiformen. Das bedeutet, daß in einem Behältnis eine Menge der Arzneiform enthalten ist, die für eine Vielzahl von Anwendungen mit entsprechenden Dosisvorgängen vorgesehen ist. Die Dosierung selbst erfolgt individuell durch den Anwender. Aussagen über die dosierte Wirkstoffmenge kann der Anwender nur machen, wenn er die jeweilige Dosis vor Anwendung wiegt. Bei wiederholter Anwendung wäre die reproduzierbare Applikation einer gleichbleibenden Wirkstoffmenge nur mit Hilfe eines vorgeschalteten Wägevorgangs möglich. Diese individuell variable Dosierung ist nur möglich aufgrund der geringen Kohärenz und leichten Abteilbarkeit dieser Arzneiformen. Andererseits bietet die geringe Kohärenz den Vorteil, daß die Arzneiform, wie erwähnt, beliebig verformbar ist und an unebene Oberflächen angepaßt werden kann.
Demgegenüber ist die erfindungsgemäße Arzneiform eine Einmaldosen-Arzneiform, die, ähnlich wie Tabletten oder Kapseln, kohärent und vorgeformt ist und eine definierte Wirkstoffdosis für eine Anwendung in homogen verteilter Form enthält. Das hat den Vorteil, daß beliebig oft und reproduzierbar eine vorbestimmte Wirkstoffmenge appliziert werden kann. Unter Kohärenz wird in diesem Zusammenhang eine Festigkeit und ein innerer Zusammenhalt der Arzneiform verstanden, die, im Gegensatz zu den dargestellten herkömmlichen Arzneiformen, eine Handhabung durch den Anwender ermöglicht, bei der die vorgegebene Arzneiformmenge und damit die gegebene Wirkstoffmenge durch die Handhabung selbst nicht automatisch bestimmt, verändert oder beeinflußt wird.
Die erfindungsgemäße Arzneiform unterscheidet sich von anderen Einmaldosen-Arzneiformen, wie beispielsweise Tabletten oder Kapseln, dadurch, daß sie zwar einerseits die zur Handhabung notwendige Kohärenz aufweist, andererseits aber flexibel und verformbar ist, so daß sie nach Einbringen in die Wunde an die Unebenheiten des Wundgrunds angepaßt und in Kontakt mit diesem gebracht werden kann. Voraussetzung dafür ist, daß die flächenförmige Ausdehnung der Arzneiform kleiner oder maximal gleich der zu versorgenden Wundfläche ist. Ähnlich wie bei den genannten festen Arzneiformen wird die Homogenität der Wirkstoffverteilung dadurch erreicht, daß zunächst eine Gesamtmasse aus den Hilfsstoffkomponenten hergestellt wird, in der Wirkstoff homogen verteilt wird. Aus einer solchen Masse wird üblicherweise im Zuge des Arzneiformungsprozesses eine Vielzahl abgeteilter Arzneiformen von gleicher Gestalt und gleichem Gewicht hergestellt, die dementsprechend alle den gleichen Wirkstoffgehalt aufweisen. Typischerweise findet im Zuge der Arzneiformung durch physikalische Mittel, z. B. Ausübung von Druck, oder chemische Reaktionen eine Verfestigung statt, die der abgeteilten, einzelnen Arzneiform ihre Kohärenz verleiht.

Zur Herstellung einer erfindungsgemäßen Arzneiform wird zunächst eine niedrigviskose, fließfähige Masse, z. B. eine Lösung, eine Dispersion oder eine Schmelze, die Wirkstoff in homogen verteilter Form enthält, hergestellt. Diese Masse wird dann nach dem Fachmann bekannten Verfahren auf ein flächiges Substrat beschichtet. Im Gegensatz zu den genannten festen Arzneiformen findet bei der Herstellung einer erfindungsgemäßen Arzneiform der Verfestigungsvorgang, der der einzelnen, abgeteilten Arzneiform ihre Kohärenz verleiht, nicht während des Arzneiformungs- und- abteilungsvorgangs statt, sondern vorher. Die Verfestigung erfolgt nach der Beschichtung auf ein flächiges Substrat durch Entzug des Lösungs- oder Dispersionsmediums mittels Trocknung bzw. durch Abkühlung, falls aus der Schmelze beschichtet wird. Der dabei stattfindende Aufbau kohäsiver Kräfte hängt in Art und Stärke von der Hilfsstoffzusammensetzung ab. Es resultiert ein breites, folienförmiges Endlosband mit einer durch die Beschichtung vorgegebenen Dicke. Limitierender Faktor für die Dicke des Bands ist bei einer gegebenen Formulierung die Forderung nach Flexibilität und Verformbarkeit der einzelnen, abgeteilten Arzneiform zur Anpassung an den Wundgrund nach Einbringen in eine Wunde. Die Abteilung einzelner Arzneiformen mit vorgegebener Fläche erfolgt aus dem Endlosband nach bekannten Verfahren, wie z. B. Stanzen und Schneiden. Da die Beschichtung mit einer Masse, die Wirkstoff in homogen verteilter Form enthält, und unter Einhaltung eines konstanten Beschichtungsgewichts durchgeführt wird, enthalten alle einzeln abgeteilten Arzneiformen die gleiche Wirkstoffmenge in homogener Verteilung. Dadurch wird dem Anwender eine exakte und reproduzierbare Dosierung ermöglicht.
Da Wirkstoffgehalt pro Flächeneinheit und Fläche selbst durch das Herstellverfahren in breitem Rahmen stufenlos variiert werden können, bietet die erfindungsgemäße Arzneiform die Möglichkeit, auch sehr geringe Wirkstoffmengen exakt und zuverlässig zu dosieren.
Darüber hinaus kann der Anwender eine an der jeweiligen Problemstellung und den Therapieerfordernissen orientierte Wirkstoffdosierung vornehmen. So kann der Anwender beispielsweise mehrere Arzneiformen gleichzeitig in die Wunde einbringen und nebeneinander auf den Wundgrund applizieren. Der Anwender kann aus einer Arzneiform gegebener Fläche aber auch kleine Stücke abteilen, wenn z.B. die zu behandelnde Wundfläche kleiner als die flächenmäßige Ausdehnung der Arzneiform ist, oder wenn die durch die Fläche gegebene Wirkstoffdosis der Arzneiform für eine spezielle Behandlung zu hoch ist. So kann die erfindungsgemäße Arzneiform dem Anwender beispielsweise zur Verfügung gestellt werden in Verbindung mit einem inerten flächenförmigen Substrat, von dem die Arzneiform sich leicht abheben läßt, wie beispielsweise einer silikonisierten Folie, wobei diese eine Einteilung in cm-Maßstab aufweisen kann. Da die Flächenbeladung der Arzneiform mit Wirkstoff bekannt ist, kann der Anwender aus einer blattförmigen oder aufgerollten, bandförmigen Arzneiform die Fläche und damit die Wirkstoffmenge aus- bzw. abschneiden, die er aus therapeutischer Sicht für notwendig erachtet.
In jedem Fall wird erreicht, daß die flächenförmige Ausdehnung der Arzneiform kleiner oder maximal gleich der zu versorgenden Wundfläche ist.Dadurch wird die Applikation an den Wundgrund ermöglicht und sichergestellt, daß die applizierte Wirkstoffmenge in der Wunde freigesetzt wird. Bei wundrandüberlappender Applikation würde nur der in die Wunde reichende Teil der Arzneiform Wirkstoff freisetzen, wodurch der Vorteil der exakten Dosierung zunichte gemacht würde.
Wirkstoffe, die in den erfindunggemäßen Arzneiformen in Wunden zur Verwendung kommen, sind vorzugsweise blutstillende Wirkstoffe, wundreinigende Wirkstoffe wie z. B. Enzyme, Antiseptika, Desinfizientia und Antibiotika sowie wundheilungsfördernde Wirkstoffe, durch die die Granulation angeregt, die Gefäßneubildung induziert oder die Epithelisierung gefördert wird. Unter den wundheilungsfördernden Wirkstoffen gewinnen biologisch aktive Peptide und Proteine, die bereits in sehr geringer Konzentration hohe Aktivitäten entfalten, und zum großen Teil mittels rekombinanter Technologien hergestellt werden, zunehmend an Bedeutung. Zu diesen Substanzen, für die die erfindungsgemäße Arzneiform ein besonders geeignetes Träger- und Abgabesystem darstellt, gehören sogenannte Wachstumsfaktoren wie Platelet derived growth factor (PDGF), Epidermal growth factor (EDF), Platelet derived endothelial cell growth factor (PD-ECGF), acidic Fibroblast growth factor (aFGF), basic Fibroblast growth factor (bFGF), Transforming growth factor a (TGFa), Transforming growth factor β (TGFβ), Keratinocyte growth factor (KGF), Insulin-like growth factors 1 und 2 (IGF1 IGF2) sowie Tumor necrosis factor (TNF).
Ein weiterer Vorteil der erfindungsgemäßen Arzneiform ist, daß aus ihr Wirkstoff kontrolliert freigesetzt werden kann. Da die Arzneiform nach Applikation in jedem Fall mit Wund-bzw. Gewebsflüssigkeit in Kontakt tritt, hat die Interaktion mit Flüssigkeit entscheidenden Einfluß auf die Wirkstofffreisetzung, was wiederum zur Steuerung der Freisetzung genützt werden kann. So kann die Rezeptur der erfindungsgemäßen Arzneiform zur Erzielung einer relativ schnellen Wirkstofffreisetzung so ausgelegt werden, daß die Arzneiform in Wundflüssigkeit löslich bzw. zerfallbar ist. Die Freisetzungskinetik für Wirkstoff hängt in diesem Fall von der Auflösungs- bzw. Zerfallsgeschwindigkeit der Arzneiform ab. Nach Ablauf der Applikationszeit muß die aufgelöste bzw. zerfallene Arzneiform, ähnlich wie Salben oder Cremes, aus der Wunde ausgewaschen werden, es sei denn, daß die Rezeptur so ausgelegt wird, daß die Arzneiform in Wundflüssigkeit bis in den molekularen Bereich der einzelnen Komponenten vollständig abbaubar und resorbierbar ist.
Eine Verzögerung und Verlängerung der Wirkstofffreisetzung kann erreicht werden, wenn die Zusammensetzung so gewählt wird, daß die Arzneiform unter Wundflüssigkeitsaufnahme lediglich quillt. Die Wundflüssigkeit löst insbesondere den Wirkstoff aus der Arzneiform heraus, was bei dieser zu einer langsamen Erosion führt. In diesem Fall hängt die Wirkstofffreisetzung vom Quellvermögen und der Erosionsgeschwindigkeit der Arzneiform ab.
Eine noch weitergehende Verzögerung und Verlängerung der Wirkstofffreisetzung wird erzielt, wenn die Zusammensetzung der Arzneiform so gewählt wird, daß diese inert gegenüber Wundflüssigkeit ist und nicht mit ihr interagiert. Die Freisetzungskinetik für Wirkstoff hängt dann nur von der Diffusionsgeschwindigkeit von Wirkstoff innerhalb der Arzneiform sowie an der Grenzfläche zwischen Arzneiform und Wundgrund bzw. Wundflüssigkeit ab.
In den genannten Fällen, in denen die Arzneiform nicht löslich oder zerfallbar ist, hat der Anwender den Vorteil, daß er diese jederzeit ohne Auswaschen oder ähnliche Manipulationen vollständig aus der Wunde entfernen kann.
In einer weiteren bevorzugten Ausführungsform ist die erfindungsgemäße Arzneiform mehrschichtig aufgebaut. So kann beispielsweise eine in Wundflüssigkeit lösliche oder zerfallbare Schicht, die der schnellen Freisetzung von Wirkstoff zur möglichst raschen Erreichung der mindest notwendigen Wirkstoffkonzentration dient, in Laminatform zusammengebracht sein mit einer quellbaren oder einer inerten Schicht, die einer langsamen und gleichmäßigen Freisetzung von Wirkstoff zur Aufrechterhaltung der notwendigen Wirkstoffkonzentration über einen längeren Zeitraum dient.
Derartige mehrschichtige Arzneiformen können auch verwendet werden, wenn beispielsweise die Freisetzung unterschiedlicher Wirkstoffe zu unterschiedlichen Zeitpunkten oder mit unterschiedlichen Freisetzungsraten erfolgen soll.
In einer bevorzugten Ausführungsform einer mehrschichtigen Arzneiform umfaßt diese ein Sperr- und/oder Steuerelement, das keinen Wirkstoff enthält, wie beispielsweise eine flexible Folie aus Polyurethan, Polyester odr Polypropylen. Mit Hilfe eines solchen Sperr- bzw. Steuerelements soll die Wirkstoffabgabe in eine bestimmte Richtung gelenkt werden. Wenn beispielsweise eine verformbare, wirkstoffabgebende Schicht an den Wundgrund appliziert wird, so kann durch eine darauf laminierte Sperrschicht verhindert werden, daß beispielsweise in einer stark exsudierenden Wunde Wirkstoff an die umgebende Wundflüssigkeit abgegeben wird, was mögklicherweise zu einem unerwünscht starken Verdünnungseffekt führen könnte. Eine erfindungsgemäße Arzneiform mit Sperrelement erweist sich beispielsweise dann von Vorteil, wenn bei Infektionen die in Wundgrundnischen sitzenden Bakterienkolonien rasch und hochkonzentriert mit Antiseptika oder Antibiotika bekämpft werden sollen.
In einer weiteren bevorzugten Ausführung der erfindungsgemäßen Arzneiform für die Wundbehandlung ist diese porös, z. B. schaum- oder schwammartig. Die Größe der Poren und die Struktur der Arzneiform ist so ausgelegt, daß das Einwandern von Zellen wie z. B. Fibroblasten in diese möglich ist und den Zellen dabei eine strukturelle Orientierung gegeben wird, die insbesondere auf den vorzugsweise dem natürlichen Bindegewebe ähnlichen Ordnungsgrad der Schwammstruktur in der Arzneiform zurückzuführen ist. Das Einwachsen der Zellen kann z.B. notwendig sein für den Abbau der Zubereitung oder zur Abgabe bzw. Ablagerung von Substanzen, die z.B. für Gewebsneubildung benötigt werden oder für die Vaskularisation eines Gewebes, das an die Stelle der erfindungsgemäßen Arzneiform nach deren Abbau treten soll. Die Voraussetzungen für die Porosität der Arzneiform würden dabei im Zuge der Herstellung dadurch geschaffen, daß beispielsweise in die zu beschichtende Masse mit homogener Verteilung von Wirkstoff Luft eingeführt wird oder daß nach Beschichtung aus der Lösung oder Dispersion durch externe Trocknungsbedingungen das verdampfende Lösemittel oder Dispersionsmedium Löcher bzw. Poren in der beschichteten Bahn hinterläßt.
Die Auswahl von Materialien und Hilfsstoffen zur Herstellung der erfindungsgemäßen Arzneiform wird zunächst bestimmt durch die Anforderung an deren Kohärenz, Flexibilität und Verformbarkeit sowie durch Anforderungen an die gewünschte Freisetzungskinetik für Wirkstoff. Weiterer beschränkender Faktor ist, daß das Spektrum verwendbarer Materialien und Hilfsstoffe auf solche, die bei Kontakt mit Wundgewebe eine ausgezeichnete Verträglichkeit aufweisen, reduziert ist, Die aus einer Kombination von Materialien und Hilfsstoffen hergestellte Arzneiform sollte nach Applikation in die Wunde Zellen wie beispielsweise Keratinocyten, Fibroblasten oder Endothelzellen in ihrer Funktion und Aktivität nicht behindern.

Mindestens notwendig zur Herstellung einer erfindungsgemäßen Arzneiform sind Hilfsstoffe aus der Gruppe der Polymere und Hilfsstoffe aus der Gruppe der Weichmacher. Polymere sorgen für den inneren Zusammenhalt und die Kohärenz der Arzneiform, da sie nach Beschichtung und Trocknung bzw. Abkühlung durch beispielsweise kovalente Bindungen, Wasserstoffbrücken oder ionische Wechselbeziehungen Netzwerke bilden, die der Verfestigung dienen und somit die notwendige Kohärenz der Arzneiform schaffen. Durch Weichmacher wird die Konsistenz der Arzneiform so eingestellt, daß sie flexibel und verformbar und somit an den Wundgrund anpaßbar ist. Geeignete Weichmacher mit physiologischer Eignung für die Wundbehandlung sind vorzugsweise niedermolekulare, mehrwertige Alkohole wie beispielsweise Glycerin, Sorbitol, niedermolekulares Polyethylenglykol oder niedermolekulares Polypropylenglykol.
Polymere mit Eignung für eine schnellfreisetzende Vorrichtung, die in Wundflüssigkeit löslich ist oder zumindest zerfällt, sind insbesondere wasserlösliche Polymere. Dazu gehören vorzugsweise Kollagen und Gelatine, pflanzliche Polysaccharide wie Alginate, Pektine, Carrageenane oder Xanthan, Cellulosederivate wie Methylcellulose, Hydroxipropylcellulose, Hydroxiethylcellulose, Hydroxipropylmethylcellulose oder Natriumcarboximethylcellulose, Stärke und Stärkederivate, Galaktomannan und Galaktomannanderivate, Chitosan und Chitosanderivate, Glykoproteine, Proteoglykane, Glucosaminoglykane, Polyvinylalkohol, Polyvinylpyrrolidon, Vinylpyrrolidon-Vinylacetat-Mischpolymerisate, höhermolekulare Polyethylenglykole und höhermolekulare Polypropylenglykole.
Polymere mit Eignung für eine verzögert und über einen längeren Zeitraum freisetzende Arzneiform, die in Wundflüssigkeit quillt oder nicht mit Wundflüssigkeit interagiert, sind insbesondere wasserquellbare oder wasserunlösliche Polymere. Dazu gehören vorzugsweise Cellulosederivate wie Ethylcellulose, Celluloseacetatphthalat, Hydroxipropylmethylcellulosephthalat, Celluloseacetatsuccinat oder Ethylcellulosesuccinat, Polyoxiethylen-Polyoxipropylen-Copolymere, Polyvinylalkohol, Polyacrylate und Polymethacrylate, Polylactide, Polyglycolide sowie Polyaminosäuren.
Als weitere Hilfsstoffe kann die Arznei enthalten:
· Konservierungsmittel, wie bspw. p-Cl-m-Kresol, Phenylethylalkohol, Phenoxiethylalkohol, Chlorbutanol, 4-Hydroxibenzoesäuremethylester, 4-Hydroxibenzoesäurepropylester, Benzalkoniumchlorid, Cetylpyridiniumchlorid, Chlorhexidindiacetat oder -digluconat, Ethanol oder Propylenglykol
· pH-Regulatoren wie bspw. Glycinpuffer, Citratpuffer, Boratpuffer, Phosphatpuffer oder Citronensäure-Phosphat-Puffer
· Antioxidantien wie bspw. Ascorbinsäure, Ascorbylpalmitat, Tocopherolacetat, Propylgallat, Butylhydroxianisol oder Butylhydroxitoluol,
· Hilfsstoffe zur Stabilisierung der biologischen Aktivität von Wirkstoffen wie Mannitol, Glucose, Lactose, Fructose, Saccharose, Cyclodextrin oder Dextran,
· Emulgierbare Hilfsstoffe wie Öle, Fette und Wachse,
· Emulsionsstabilatoren wie z.B. nichtionogene Emulgatoren, amphotere Emulgatoren, kationaktive Emulgatoren und anionaktive Emulgatoren,
· Füllstoffe wie z.B. mikrokristalline Cellulose, Aluminiumoxid, Zinkoxid, Titandioxid, Talkum, Siliciumdioxid, Magnesiumsilikat, Magnesium-Aluminiumsilikat, Kaolin, hydrophobe Stärke, Calciumstearat oder Calciumphosphat,
· Schäumungsmittel wie Saponine, Alginsäureester, Aminoxide oder Fettaminoxide

### Beispiel 1:

34 g Aceton, 6 g Polyethylenglycol 400 und 16 g Ethylacetat werden in einem verschließbaren Rührgefäß vorgelegt. In dem Lösemittelgemisch werden unter gleichmäßigem Rühren nacheinander 33,6 g eines Polyvinylpyrrolidon-Polyvinylacetat-Copolymers, 2 g eines Polyoxyethylen-Polyoxypropylen-Copolymers und 3,3 g Hydroxipropylcellulose gelöst.

### Beispiel 2:

In der Stammlösung nach Beispiel 1 werden unter gleichmäßigem Rühren 3,3 g Lidocain gelöst. Die Lösung wird mit einer Strichstärke von 300 µm auf ein silikonisiertes Papier gestrichen und konvektiv in einem Trockenkanal bei 50°C und einer Luftgeschwindigkeit von ca. 5 m/sec getrocknet. Nach dem Trocknen wird ein weicher, leicht getrübter, flexibler Film erhalten, der ein Flächengewicht von 130 g/m² und dementsprechend einen Wirkstoffgehalt von 0,8 mg Lidocain/cm² aufweist.
Aus dem Film werden runde Arzneiformen mit einer Fläche von 5 cm² und dementsprechend einem Wirkstoff von 4 mg Lidocain ausgestanzt. Die Arzneiformen werden jeweils in eine Paddle-Over-Disk-Apparatur gegeben und in 500 ml entmineralisiertem Wasser bei 32°C mit 50 U/min gerührt. Nach 30 Minuten, 1, 2, 4 und 24 Stunden werden jeweils 10 ml des Freisetzungsmediums entnommen. Die freigesetzte Wirkstoffmenge wird mittels HPLC bestimmt. Ergebnis:

| Entnahmezeitpunkt | freigesetzte Wirkstoffmenge (in mg; kumuliert) |
|---|---|
| 30 Min | 3,1865 |
| 1 Stunde | 3,4828 |
| 2 Stunden | 3,7978 |
| 4 Stunden | 3,8807 |
| 24 Stunden | 4,1102 |

Nach 30 Min ist die Arzneiform in mehrere kleine Teile zerfallen; zu diesem Zeitpunkt sind bereits knapp 80% und nach 4 Stunden 97% des Wirkstoffs aus der Arzneiform freigesetzt. Die gewünschten Anforderungen an diese Art von Arzneiform, schnelle Wirkstofffreisetzung zur Erzielung einer raschen Schmerzlinderung und möglichst vollständige Nutzung des applizierten Wirkstoffs, werden erfüllt.

### Beispiel 3:

In der Stammlösung nach Beispiel 1 werden unter gleichmäßigem Rühren 5,2 g Chlorhexidinhydrochlorid gelöst. Die Lösung wird unter gleichen Bedingungen wie in Beispiel 2 auf ein silikonisiertes Papier gestrichen und getrocknet. Der resultierende weiche, verformbare Film weist ein Flächengewicht von 130 g/m² und dementsprechend einen Wirkstoffgehalt von 1,2 mg Chlorhexidinhydrochlorid/cm2 auf. Aus dem Film werden runde Arzneiformen mit einer Fläche von 5 cm² und dementsprechend einem Wirkstoffgehalt von 6 mg Chlorhexidinhydrochlorid ausgestanzt. Die Arzneiformen werden jeweils in eine Paddle-Over-Disk-Apparatur gegeben und in 500 ml entmineralisiertem Wasser bei 32°C mit 50 U/min gerührt. Nach 30 Minuten und einer Stunde werden jeweils 10 ml des Freisetzungsmediums entnommen. Die freigesetzte Wirkstoffmenge wird mittels HPLC bestimmt. Ergebnis:

| Entnahmezeitpunkt | freigesetzte Wirkstoffmenge (in mg; kumuliert) |
|---|---|
| 30 Minuten | 6,1400 |
| 1 Stunde | 6,1963 |

Nach 30 Minuten ist die Arzneiform vollständig zerfallen, und es sind 100 % der applizierten Wirkstoffmenge freigesetzt. Bei Anwendung würde die antiseptische Wirkung des Chlorhexidinhydrochlorids sehr schnell und hochkonzentriert einsetzen, was bei Infektionen auch erforderlich ist. Die Ausnutzung des eingebrachten Wirkstoffs ist optimal.

### Beispiel 4:

34 g Aceton, 16 g Ethylacetat und 6 g Polyethylenglykol 400 werden in einem verschließbaren Rührgerät vorgelegt. In dem Lösemittelgemisch werden unter gleichmäßigem Rühren nacheinander 6 g Ethylcellulose, 11 g eines Polyvinylpyrrolidon-Polyvinylacetat-Copolymers, 5,5 g Hydroxipropylcellulose, 1 g Polyoxiethylen-Polyoxipropylen-Copolymers und 0,9 g Estradiol gelöst.
Die Lösung wird mit einer Strichstärke von 400 µm auf ein silikonisiertes Papier gestrichen und konvektiv in einem Trockenkanal bei 50°C und einer Luftgeschwindigkeit von ca. 5m/sec getrocknet. Nach dem Trocknen wird ein weicher, verformbarer Film erhalten, der ein Flächengewicht von 130 g/m² und dementsprechend einen Wirkstoffgehalt von 0,385 mg Estradiol/cm ² aufweist. Dieser Film unterscheidet sich von den in den Beispielen 2 und 3 beschriebenen dadurch, daß es zur Erzielung einer wundheilungsfördernden Wirkung durch Applikation von Estradiol notwendig ist, daß von diesem Wirkstoff optimalerweise nach Freisetzung einer Initialdosis kontinuierlich und kontrolliert über einen längeren Zeitraum niedrig dosiert eine Erhaltungsdosis freigesetzt werden muß.
Zur Untersuchung der Freisetzungskinetik werden aus dem Film runde Arzneiformen mit einer Fläche von 5 cm² und einem Wirkstoffgehalt von 1,924 mg Estradiol ausgestanzt. Die Vorrichtungen werden jeweils in eine Paddle-Over-Disk-Apparatur gegeben und in 500 ml entmineralisiertem Wasser bei 32°C mit 50 U/min gerührt. Nach 30 Minuten sowie 2, 6 und 24 Stunden werden jeweils 10 ml des Freisetzungsmediums entnommen. Die freigesetzte Wirkstoffmenge wird mittel HPLC bestimmt.

### Ergebnis:

| Entnahmezeitpunkt | freigesetzte Wirkstoffmenge ( in mg, kumuliert) |
|---|---|
| 30 Minuten | 0,9957 |
| 2 Stunden | 1,0943 |
| 6 Stunden | 1,1814 |
| 24 Stunden | 1,3079 |

Nach 30 Minuten ist die Arzneiform mäßig gequollen; es ist etwa die Hälfte der applizierten Wirkstoffmenge freigesetzt. Die Ergebnisse zeigen, daß danach die Wirkstofffreisetzung deutlich reduziert wird und als Erhaltungsdosis ca. 10 - 20 µg Estradiol pro Stunde abgegeben werden. Nach 24 Stunden sind 68 % der applizierten Wirkstoffmenge freigesetzt. Das Wirkstoffreservoir der Arzneiform, die auch nach mehreren Stunden zerfällt, ist so ausreichend bemessen, daß auch bei mehrtägiger Anwendung eine kontinuierliche, niedrig dosierte Estadiol-Freisetznug möglich ist.

## Patentansprüche

1. Einmaldosen-Arzneiform zur Abgabe von Wirkstoffen an eine Wundoberfläche, dadurch gekennzeichnet, daß diese als auf einem filmartigen flexiblen Substrat angeordnete vorportionierte plastische kohärente Masse mit definiertem Wirkstoffgehalt in homogener Verteilung ausgebildet ist und auf der dem Substrat abgewandten Seite eine an die Wundoberfläche andrückbare Kontaktfläche für eine lückenlose Wirkstoffabgabe an die kontaktierte Wundoberfläche aufweist.

2. Arzneiform nach Anspruch 1, dadurch gekennzeichnet, daß sie mehrteilig und in mehreren kleinen Teilen in die Wunde einbringbar ist.

3. Arzneiform nach Anspruch 1, dadurch gekennzeichnet, daß sie einteilig und vor Applikation zur Ermöglichung des Einbringens in die Wunde individuell auf die Fläche der jeweiligen Wunde zuschneidbar ist.

4. Arzneiform nach einem oder mehreren der Ansprüche 1 bis 3, dadurch gekennzeichnet, daß sie mit Mitteln zur kontrollierbaren Freisetung vom Wirkstoff ausgebildet ist.

5. Arzneiform nach einem oder mehreren der Ansprüche 1 bis 4, dadurch gekennzeichnet, daß sie in Wundflüssigkeit löslich bzw. zerfallbar ist, wobei die Freisetzungskinetik für Wirkstoff von ihrer Auflösungs- bzw. Zerfallsgeschwindigkeit abhängt.

6. Arzneiform nach einem oder mehreren der Ansprüche 1 bis 5, dadurch gekennzeichnet, daß sie in Wundflüssigkeit abbaubar und resorbierbar ist.

7. Arzneiform nach einem oder mehreren der Ansprüche 1 bis 6, dadurch gekennzeichnet, daß sie in Wundflüssigkeit quellbar ist, wobei die Freisetzungskinetik für den Wirkstoff von der Erosionsgeschwindigkeit der Arzneiform abhängt.

8. Arzneiform nach einem oder mehreren der Ansprüche 1 bis 7, dadurch gekennzeichnet, daß sie gegenüber Wundflüssigkeit inert ist und die Freisetzungskinetik für Wirkstoff nur von der Diffusionsgeschwindigkeit des Wirkstoffs abhängt.

9. Arzneiform nach einem oder mehreren der Ansprüche 1 bis 8, dadurch gekennzeichnet, daß sie einen mehrschichtigen Aufbau aufweist.

10. Arzneiform nach einem oder mehreren der Ansprüche 1 bis 9, dadurch gekennzeichnet, daß sie zur Lenkung der Wirkstoffabgabe in eine bestimmte Richtung mindestens ein Sperr- und/oder Steuerelement aufweist.

11. Arzneiform nach einem oder mehreren der Ansprüche 1 bis 10, dadurch gekennzeichnet, daß sie mindestens einen Hilfsstoff aus der Gruppe der die Freisetzungsgeschwindigkeit beeinflussenden Polymere und Hilfsstoffe aus der Gruppe der Weichmacher enthält.

12. Arzneiform nach einem oder mehreren der Ansprüche 1 bis 11, dadurch gekennzeichnet, daß sie wasserlösliche Polymere enthält.

13. Arzneiform nach Anspruch 11, dadurch gekennzeichnet, daß sie wasserquellbare und/oder wasserunlösliche Polymere enthält.

14. Verfahren zur Herstellung einer Arzneiform nach Anspruch 1 zur Abgabe von Wirkstoff an Wunden, dadurch gekennzeichnet, daß zunächst eine niedrigviskose, fließfähige Masse, eine Lösung, eine Dispersion oder eine Schmelze, die Wirkstoffe in homogen verteilter Form enthält, zubereitet und auf ein flächiges Substrat beschichtet wird, wonach eine Verfestigung der Masse durch Entzug des Lösungs- oder Dispersionsmediums mittels Trocknung oder bei Schmelze mittels Abkühlung vorgenommen wird, wobei ein folienförmiges Flächenmaterial mit einer durch die Beschichtung vorgegebenen Dicke resultiert, woraus zuletzt durch Stanzen oder Schneiden eine Anzahl von Arzneiformen von gleicher Gestalt und gleichem Gewicht abgeteilt werden.

## Claims

1. Single-dose pharmaceutical form for delivery of active substances to the surface of a wound, characterized in that it is formed as a pre-portioned coherent deformable composition having a defined content of active substance(s) in homogeneously dispersed form, said composition being arranged on a film-like flexible substrate, and in that it has on the side which is averted from the substrate a contact surface which can be pressed onto the wound surface for uninterrupted delivery of active substance(s) to the wound surface being in contact with said pharmaceutical form.

2. Pharmaceutical form according to Claim 1, characterized in that it consists of several parts and can be introduced into the wound as several small parts.

3. Pharmaceutical form according to Claim 1, characterized in that it is in one part and can, before administration, be cut individually to fit the area of the particular wound, in order to make introduction into the wound possible.

4. Pharmaceutical form according to one or more of Claims 1 to 3, characterized in that it is designed with means for controllabel release of an active substance.

5. Pharmaceutical form according to one or more of Claims 1 to 4, characterized in that it is soluble or able to disintegrate in wound fluid, with the kinetics of release of active substance depending on its rate of dissolution or disintegration.

6. Pharmaceutical form according to one or more of Claims 1 to 5, characterized in that it can be broken down and absorbed in wound fluid.

7. Pharmaceutical form according to one or more of Claims 1 to 6, characterized in that it is able to swell in wound fluid, with the kinetics of release of the active substance depending on the rate of erosion of the pharmaceutical form.

8. Pharmaceutical form according to one or more of Claims 1 to 7, characterized in that it is inert to wound fluid, and that the kinetics of release of active substance depend only on the rate of diffusion of the active substance.

9. Pharmaceutical form according to one or more of Claims 1 to 8, characterized in that it has a multilayer structure.

10. Pharmaceutical form according to one or more of Claims 1 to 9, characterized in that it has, to direct the delivery of active substance in a particular direction, at least one barrier and/or control element.

11. Pharmaceutical form according to one or more of Claims 1 to 10, characterized in that it contains at least one ancillary substance from the group of polymers influencing the rate of release and ancillary substances from the group of plasticizers.

12. Pharmaceutical form according to one or more of Claims 1 to 11, characterized in that it contains water-soluble polymers.

13. Pharmaceutical form according to Claim 11, characterized in that it contains polymers which are swellable in water and/or insoluble in water.

14. Process for the production of a pharmaceutical form according to Claim 1 for the delivery of active substance to wounds, characterized in that first a low-viscosity, free-flowing composition, a solution, a dispersion, or a melt, which contains active substances in homogeneously dispersed form, is prepared and is coated onto a flat substrate, after which the composition is consolidated by removing the dissolving or dispersing medium by drying or, in the case of a melt, by cooling, resulting in a sheet-like flat material with a thickness which is predetermined by the coating, from which finally a number of pharmaceutical forms of the same shape and same weight are divided out by punching or cutting.

## Revendications

1. Forme médicamenteuse à dose unitaire pour l'apport de principes actifs à la surface d'une blessure, caractérisée en ce que celle-ci est formée d'une masse plastique cohérente préportionnée disposée sur un substrat flexible de type film, avec une teneur définie en principes actifs à distribution homogène et en ce qu'elle présente sur la face opposée au substrat une surface de contact pouvant être pressée contre la surface de la blessure en vue d'un apport sans lacunes des principes actifs à la surface de la blessure en contact.

2. Forme médicamenteuse selon la revendication 1, caractérisée en ce qu'elle est constituée de plusieurs parties et en ce qu'elle peut être introduite dans la blessure en plusieurs petites parties.

3. Forme médicamenteuse selon la revendication 1, caractérisée en ce qu'elle est constituée d'une seule partie et en ce qu'elle peut être découpée individuellement d'après la surface de la blessure respective avant l'application pour permettre son introduction dans la blessure.

4. Forme médicamenteuse selon une ou plusieurs des revendications 1 à 3, caractérisée en ce qu'elle est munie de moyens pour la libération contrôlable du principe actif.

5. Forme médicamenteuse selon une ou plusieurs des revendications 1 à 4, caractérisée en ce qu'elle est soluble, respectivement dégradable dans l'exsudat de la blessure, la cinétique de libération du principe actif étant fonction de sa vitesse de dissolution, respectivement de dégradation.

6. Forme médicamenteuse selon une ou plusieurs des revendications 1 à 5, caractérisée en ce qu'elle est décomposable, respectivement résorbable dans l'exsudat de la blessure.

7. Forme médicamenteuse selon une ou plusieurs des revendications 1 à 6, caractérisée en ce qu'elle est gonflable dans l'exsudat de la blessure, la cinétique de libération du principe actif étant fonction de la vitesse d'érosion de la forme médicamenteuse.

8. Forme médicamenteuse selon une ou plusieurs des revendications 1 à 7, caractérisée en ce qu'elle est inerte face à l'exsudat de la blessure et en ce que la cinétique de libération du principe actif n'est fonction que de la vitesse de diffusion du principe actif.

9. Forme médicamenteuse selon une ou plusieurs des revendications 1 à 8, caractérisée en ce qu'elle présente une structure multicouche.

10. Forme médicamenteuse selon une ou plusieurs des revendications 1 à 9, caractérisée en ce qu'elle présente au moins un élément de barrage et/ou de contrôle pour l'orientation de la libération de principe actif dans une direction déterminée.

11. Forme médicamenteuse selon une ou plusieurs des revendications 1 à 10, caractérisée en ce qu'elle contient au moins un adjuvant du groupe des polymères influençant la vitesse de libération et des adjuvants du groupe des plastifiants.

12. Forme médicamenteuse selon une ou plusieurs des revendications 1 à 11, caractérisée en ce qu'elle contient des polymères solubles dans l'eau.

13. Forme médicamenteuse selon la revendication 11, caractérisée en ce qu'elle contient des polymères gonflables dans l'eau et/ou insolubles dans l'eau.

14. Procédé de préparation d'une forme médicamenteuse selon la revendication 1 pour l'apport de principe actif à des blessures, caractérisé en ce qu'on prépare d'abord une masse fluide de basse viscosité, une solution, une dispersion ou une masse en fusion, qui contient les principes actifs distribués de manière homogène, et en ce qu'on l'étend sur un substrat plan, en ce qu'on procède ensuite à la solidification de la masse par retrait du milieu de solution ou de dispersion par séchage ou, dans le cas d'une masse en fusion, par refroidissement, entraînant un matériau plan sous forme d'un film d'une épaisseur prédéterminée par le revêtement et en ce qu'on le divise finalement par poinçonnage ou par découpe en un nombre de formes médicamenteuses de même aspect et de même poids.
